# EUROPÄISCHE PATENTSCHRIFT

(11) **EP 1 353 759 B1**
(45) Veröffentlichungstag und Bekanntmachung des Hinweises auf die Patenterteilung: **16.01.2008**
(21) Anmeldenummer: 02719714.4
(22) Anmeldetag: 23.01.2002
(51) Int. Cl.: B05B 17/00

(54) **AEROSOLGENERATOR**
AEROSOL GENERATOR
DISPOSITIF DE PRODUCTION D'AEROSOL

(30) Priorität: 23.01.2001 DE 10102846
(43) Veröffentlichungstag der Anmeldung: 22.10.2003
(73) Patentinhaber: PARI Pharma GmbH, 82319 Starnberg (DE)
(72) Erfinder: GALLEM, Thomas, 81371 München (DE); URICH, Markus, 81379 München (DE)
(74) Vertreter: HOFFMANN EITLE
(86) Internationale Anmeldenummer: PCT/EP2002/000648
(87) Internationale Veröffentlichungsnummer: WO 2002/064265

(56) Entgegenhaltungen:
- EP-A- 1 205 199
- WO-A-00/47334
- WO-A-97/29851
- US-A- 6 068 198

## Beschreibung

Die Erfindung betrifft Aerosolgeneratoren, bei denen mit Hilfe einer Schwingmembran eine Flüssigkeit zerstäubt wird, insbesondere zur Anwendung in Aerosoltherapiegeräten.

Aerosolgeneratoren dieser Art besitzen ein Flüssigkeitsreservoir, in dem die für die Zerstäubung vorgesehene Flüssigkeit bevorratet wird. Die Flüssigkeit wird einer Membran zugeführt, die in Schwingungen versetzt wird. Die auf der einen Seite der Schwingmembran anstehende Flüssigkeit wird dabei durch Öffnungen in der Schwingmembran hindurch transportiert und liegt auf der anderen Seite der Schwingmembran als Aerosol vor.

In WO 97/29851, in der ein Aerosolgenerator dieser Art beschrieben ist, wird ausgeführt, dass Aerosolgeneratoren dieser Art besonders effektiv arbeiten, wenn die Flüssigkeit bei einem Druck zugeführt wird, der etwas unterhalb des Umgebungsdrucks des Bereichs liegt, in den die von der Membran erzeugten Tröpfchen des Aerosols abgegeben werden.

Um die Erzeugung des Aerosols wirksam und planmäßig zu unterstützen, muss der Unterdruck insbesondere dann in vorgegebenen Grenzen erzeugt werden, wenn mit dem Aerosolgenerator ein Medikament in einem Aerosoltherapiegerät vernebelt wird. Denn die Einhaltung vorgegebener Parameter ist entscheidend für die Tröpfchengröße und die Output-Rate des Aerosols und damit für die therapeutische Anwendung und Wirksamkeit, da diese Größen vorrangig die Dosierung und Deposition der Aerosolpartikel in der Lunge des Patienten bestimmen.

Daneben muss eine Aerosoltherapievorrichtung mit Membran-Aerosolgenerator als Ganzes für den Patienten einfach zu bedienen sein, was auch für die Erzeugung bzw. Einstellung des Unterdrucks.im Flüssigkeitsreservoir des Aerosolgenerators gilt.

Vor diesem Hintergrund besteht das der Erfindung zugrunde liegende Problem darin, einen Aerosolgenerator mit einer Vorrichtung zur Erzeugung eines Unterdrucks im Flüssigkeitsreservoir des Aerosolgenerators anzugeben, der die eingangs geschilderten Anforderungen erfüllt.

Im folgenden wird die Erfindung anhand eines Ausführungsbeispiels unter Bezugnahme auf die Figuren näher beschrieben, in denen zeigt:
- Fig. 1: ein Aerosoltherapiegerät mit einem erfindungsgemäßen Aerosolgenerator;
- Fig. 2: den erfindungsgemäßen Aerosolgenerator gemäß Fig. 1 in vergrößerter Darstellung;
- Fig. 3: eine Draufsicht auf die Handhabe des erfindungsgemäßen Aerosolgenerators; und
- Fig. 4: eine Draufsicht auf die Lagerhülse des erfindungsgemäßen Aerosolgenerators aus Fig. 2.

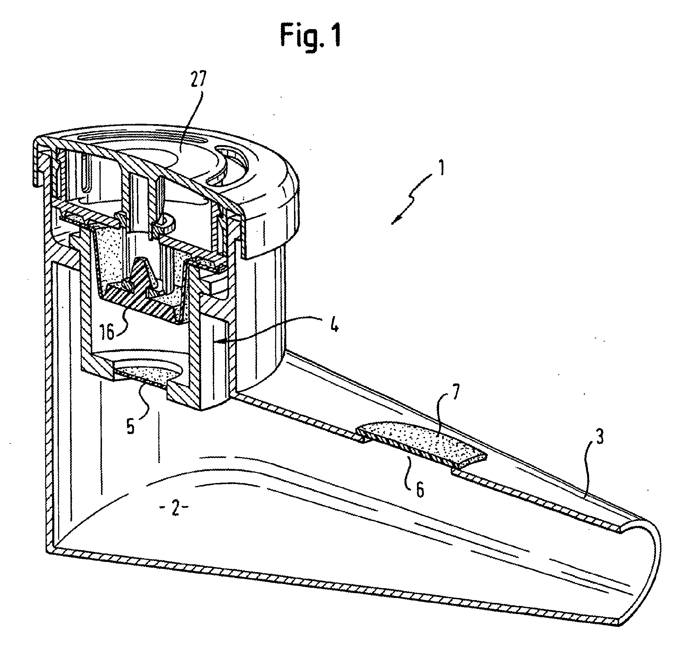

Fig. 1 zeigt ein Aerosoltherapiegerät 1, mit einem Verneblungsraum 2, einem Mundstück 3 und einem Membran-Aerosolgenerator 4, dessen Schwingmembran in Fig. 1 mit 5 gekennzeichnet ist. Die Schwingmembran kann z.B. durch ringförmige Piezo-Elemente (nicht dargestellt) in Schwingungen versetzt werden, was unter anderem in WO 97/29851 beispielhaft beschrieben ist. Auf der einen Seite der Schwingmembran 5, in Fig. 1 oben, befindet sich im Gebrauchsfall die Flüssigkeit, die durch Öffnungen in der Schwingmembran 5 hindurch transportiert und auf der anderen Seite der Schwingmembran 5, in Fig. 1 unten, als Aerosol in den Verneblungsraum 2 abgegeben wird. Der Patient kann an dem Mundstück 3, das im Verneblungsraum 2 vorhandene Aerosol abatmen. Um den Patienten nicht nach dem Einatmen des Aerosols zum Absetzen des Therapiegeräts zu veranlassen, besitzt das Mundstück 3 eine Öffnung 6, die von einem elastischen Ventilelement 7 verschlossen ist. Atmet der Patient in das Mundstück 3 und damit in den Verneblungsraum 2 hinein aus, öffnet sich das elastische Ventilelement 7, so dass die Ausatemluft aus dem Inneren des Aerosoltherapiegeräts austreten kann. Beim Einatmen strömt Umgebungsluft durch den Aerosolgenerator 4 hindurch, was im folgenden noch genauer beschrieben wird.

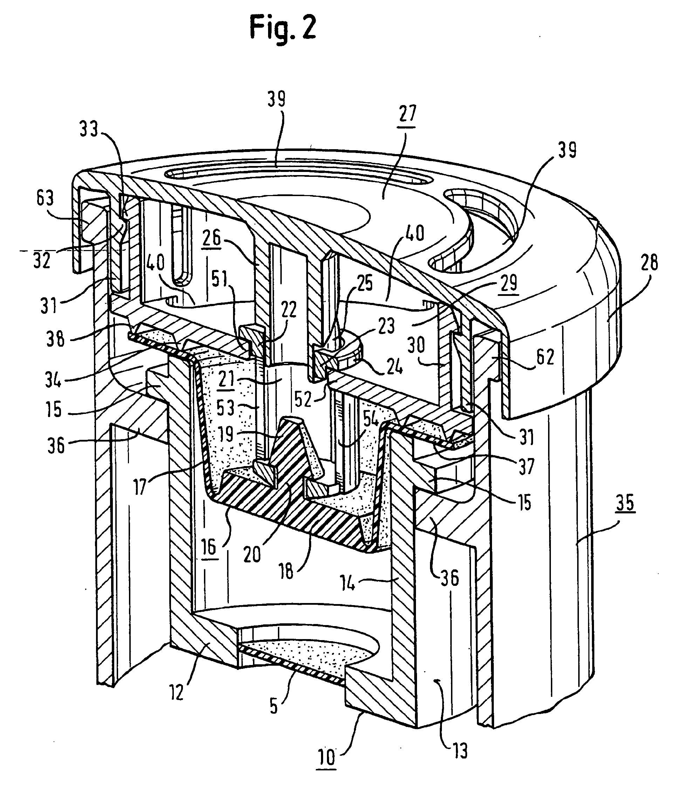

Zunächst wird im folgenden unter Bezugnahme auf Fig. 2 jedoch der Aufbau des erfindungsgemäßen Aerosolgenerators dargestellt.

Der hier beispielhaft beschriebene Aerosolgenerator gemäß Fig. 2 umfasst einen zylindrischen Vorratsbehälter 10 zur Bevorratung einer Flüssigkeit, die der Membran 5 zugeführt wird. Die Schwingmembran 5 kann, wie in Fig. 2 gezeigt, in einer Stirnwand 12 des zylindrischen Flüssigkeitsreservoirs 10 angeordnet sein, so dass die in das Flüssigkeitsreservoir eingefüllte Flüssigkeit unmittelbar mit der Membran 5 in Berührung kommt, wenn der erfindungsgemäße Aerosolgenerator in der in Fig. 1 gezeigten Stellung gehalten wird. Jedoch sind auch andere Zuführungen für die Flüssigkeit zu der Schwingmembran einsetzbar, ohne dass die Gestaltung der erfindungsgemäßen Vorrichtung zur Erzeugung eines Unterdrucks im Flüssigkeitsreservoir verändert werden muss. Aufgrund der kompakten Bauform des Aerosolgenerators gemäß Fig. 1 bzw. 2 ist diese Ausführung aber besonders vorteilhaft.

Auf der der Stirnwand 12 gegenüberliegenden Seite ist der zylindrische Flüssigkeitsbehälter 10 offen. Die Öffnung dient dem Einfüllen der Flüssigkeit in das Flüssigkeitsreservoir 10. Ein wenig unterhalb der Öffnung läuft auf der äußeren Oberfläche 13 der Umfangswand 14 ein Vorsprung 15 um, der als Auflage dient, wenn der Flüssigkeitsbehälter in eine entsprechend ausgebildete Öffnung in einem Gehäuse 35 eingesetzt wird.

Verschlossen wird die offene Stirnseite des Flüssigkeitsbehälters 10 durch ein flexibles Dichtungselement 16. Das Dichtungselement 16 liegt auf der Stirnfläche der Umfangswand 14 des Flüssigkeitsbehälters 10 auf und erstreckt sich topfförmig in das Innere des Flüssigkeitsbehälters 10, indem ein konisch verlaufender Wandabschnitt 17 in dem Dichtungselement 16 ausgebildet ist und von einem ebenen Wandabschnitt 18 des Dichtungselements 16 abgeschlossen wird. Wie weiter unten noch erläutert wird, wirken über den ebenen Wandabschnitt 18 Kräfte auf das Dichtungselement 16 ein, so dass der ebene Wandabschnitt 18 vorzugsweise dicker ist als die übrigen Abschnitte des Dichtungselements 16. Am Umfang des ebenen Wandabschnitts 18 ist zum konischen Wandabschnitt 17 hin ein Abstand vorgesehen, so dass sich der konische Wandabschnitt 17 in Falten legen kann, wenn der ebene Wandabschnitt 18 - bezogen auf die Darstellung in Fig. 2 - nach oben bewegt wird.

Auf der dem Innenraum des Flüssigkeitsbehälters abgewandten Seite des ebenen Wandabschnitts 18 ist ein Vorsprung vorgesehen, der über einen kegelstumpfförmigen Abschnitt 19 und einen zylindrischen Abschnitt 20 verfügt. Diese Gestaltung erlaubt das Einführen und Einrasten des Vorsprungs in einer an den zylindrischen Abschnitt angepassten Öffnung, da das flexible Material des Dichtungselements 16 eine Verformung des kegelstumpfförmigen Abschnitts 19 erlaubt.

Erfindungsgemäß umfasst der Aerosolgenerator 4 eine mit einer solchen Öffnung ausgestattete Zughülse 21, bei der es sich im wesentlichen um einen einseitig offenen Hohlzylinder handelt. Die Öffnung für die Befestigung des Dichtungselements 16 ist in einer Stirnwand der Zughülse 21 ausgebildet. Nach dem Einrasten des Kegelstumpfs 19 liegt die die Öffnung enthaltende Stirnwand der Zughülse 21 auf dem ebenen Dichtungselementwandabschnitt 18 auf. Durch die Einrastung des Kegelstumpfs 19 in der Zughülse ist es möglich, dass Kräfte von der Zughülse 21 auf den ebenen Wandabschnitt 18 des Dichtungselements 16 übertragen werden, so dass der Dichtungsabschnitt 18 den Bewegungen der Zughülse 21 in Richtung der Mittellängsachse des Flüssigkeitsbehälters 10 folgt.

Die Zughülse 21 kann in verallgemeinerter Form angesehen werden als Zugelement, das beispielsweise auch durch eine aufgeklebte oder durch eine Bohrung gesteckte Zugstange realisiert sein kann. Charakterisierend für das Zugelement 21 ist, das mit seiner Hilfe eine im wesentlichen geradlinig gerichtete Kraft auf das ebene Wandelement 18 des Dichtungselements 16 übertragen wird. Insgesamt ist entscheidend für die Funktionsweise des erfindungsgemäßen Aerosolgenerators, dass ein Zugelement eine geradlinige Bewegung auf das Dichtungselement überträgt, so dass eine Volumenvergrößerung innerhalb des Flüssigkeitsreservoirs 10 eintritt. Damit wird erreicht, da das Flüssigkeitsreservoir 10 ansonsten gasdicht ausgestaltet ist, dass in dem Flüssigkeitsreservoir 10 ein Unterdruck entsteht.

Das Dichtungselement 16, das Zugelement 21 können einstückig, d.h. in einem Arbeitsvorgang, aber aus unterschiedlichen Materialien gefertigt werden. Die Fertigungstechnologie dafür steht zur Verfügung, so dass eine einstückig handhabbare Komponente des erfindungsgemäßen Aerosolgenerators entsteht, die in einem voll automatisierten Herstellungsschritt gefertigt werden kann.

Die Zughülse 21 ist auf der der Bohrung für den Kegelstumpf gegenüberliegenden Stirnseite offen, jedoch ragen zumindest zwei vorzugsweise diametral gegenüberliegende Nasen 22 und 23 radial in den Innenraum der Zughülse 21 hinein. Radial nach aussen erstreckt sich ein um die Zughülse umlaufender Kragen 24. Während der Kragen 24 als Auflage der Zughülse 21 in der in Fig. 2 gezeigten Stellung dient, übernehmen die in den Innenraum der Zughülse 21 hineinragenden Vorsprünge 22 und 23 die Aufgabe, insbesondere parallel zur Mittellängsachse wirkende Kräfte auf die Zughülse 21 aufzunehmen. Erfindungsgemäß werden diese Kräfte hervorgerufen mit Hilfe von zwei spiralförmigen Nuten 25, die aussen in der Umfangswand einer Drehhülse 26 ausgebildet sind.

Die erfindungsgemäße Vorrichtung kann auch mit einem der Vorsprünge 22 oder 23 und einer Nut 25 realisiert werden. Jedoch ist die gleichmäßig verteilte Anordnung von zwei oder mehr Vorsprüngen und einer entsprechenden Anzahl von Nuten zu bevorzugen.

Auch bei der Drehhülse 26 handelt es sich um einen einseitig offenen Zylinder, wobei das offene Stirnende in der Zughülse 21 und damit dem Kegelstumpf 19 zugewandt angeordnet ist, was ein Eindringen des Kegelstumpfes 19 in die Drehhülse 26 gestattet. Die Drehhülse 26 ist ferner so in der Zughülse 21 angeordnet, dass die Vorsprünge 22 und 23 in den spiralförmigen Nuten 25 liegen. Die Steigung der spiralförmigen Nut 25 ist derart gestaltet, dass beim Verdrehen der Drehhülse 26 in Bezug auf die Zughülse 21 die Vorsprünge 22 und 23 in den spiralförmigen Nuten 25 entlang gleiten, wodurch eine parallel zur Mittellängsachse gerichtete Kraft auf die Gleitvorsprünge 22 und 23 und damit auf die Zughülse 21 ausgeübt wird. Die Zughülse 21 wird durch diese Kraft in Richtung der Mittellängsachse verschoben, so dass auch das mit Hilfe des Kegelstumpfs in der Bohrung der Zughülse eingerastete Dichtungselement 16 im wesentlichen parallel zur Mittellängsachse verschoben wird.

Durch die Verschiebung des Dichtungselements 16 in Richtung der Mittellängsachse des Flüssigkeitsbehälters 10 wird ein Unterdruck im Flüssigkeitsbehälter 10 erzeugt, der unter anderem von der Strecke bestimmt wird, um die die Zughülse 21 in Richtung der Mittellängsachse verschoben wird. Durch die Verschiebung wird das Volumen des gasdichten Flüssigkeitsbehälters 10 vergrößert und dadurch ein Unterdruck erzeugt. Diese Verschiebung wiederum ist festgelegt durch die Gestaltung der spiralförmigen Nuten 25 in der Drehhülse 26. Somit wird bei dem erfindungsgemäßen Aerosolgenerator erreicht, dass der Unterdruck im Flüssigkeitsreservoir 10 in den relevanten Bereichen durch einfache konstruktive Maßnahmen einstellbar wird.

Damit die bei der Handhabung der Vorrichtung zur Erzeugung des Unterdrucks aufzubringenden Kräfte gering bleiben, ist die Drehhülse 26 einstückig ausgebildet mit einer Handhabe 27, deren Größe so gewählt ist, dass ein Verdrehen den Handhabe 27 und damit der Drehhülse 26 durch den Benutzer von Hand ohne große Anstrengung erfolgen kann. Die Handhabe 27 besitzt im wesentlichen die Form eines einseitig offenen, flachen Zylinders oder Kegelstumpfs, so dass sich am äußeren Umfang der Handhabe 27 ein umlaufender Griffbereich 28 ergibt, den der Benutzer beim Verdrehen der Handhabe 27 mit der Hand berührt. Auf Grund der Gestaltung der spiralförmigen Nuten 25 und des insgesamt vergleichsweise kurzen Weges, den die Zughülse 21 in Längsrichtung zurückzulegen hat, um einen ausreichenden Unterdruck zu erzeugen, ist es nur erforderlich, die Handhabe 27 und damit die Drehhülse 26 um einen vergleichsweise kleinen Winkel zu drehen. In bevorzugten Ausgestaltungen liegt dieser Verdrehwinkel in einem Bereich von 45° bis 360°. Diese Gestaltung trägt in besonderem Maße zu der einfachen Handhabbarkeit der erfindungsgemäßen Vorrichtung und eines damit ausgerüsteten Aerosolgenerators bzw. Aerosoltherapiegeräts bei.

Um aus der Zughülse 21 und der Drehhülse 26 einschließlich Handhabe 27 eine einfach und einheitlich handhabbare Einheit zu machen, ist bei dem hier beschriebenen Ausführungsbeispiel des erfindungsgemäßen Aerosolgenerators eine Lagerhülse 29 für die Lagerung der Zughülse 21 vorgesehen, bei der es sich im wesentlichen um einen flachen einseitig offenen Zylinder handelt. Der Durchmesser der Umfangswand 30 der Lagerhülse 29 ist kleiner als der Innendurchmesser der Handhabe 27 und ist bei dem beschriebenen Ausführungsbeispiel ausgerichtet am Innendurchmesser eines zylindrischen Rastringes 31, der konzentrisch zum Griffbereich 28 der Handhabe 27, aber mit kleinerem Durchmesser auf der Seite der Handhabe 27 vorgesehen ist, auf der auch die Drehhülse 26 angeordnet ist. Auf der der Drehhülse zugewandten Seite des zylindrischen Rastringes 31 ist eine umlaufende Rastkante 32 ausgebildet, die mit abschnittsweise ausgebildetem Rastnasen 33 an der Umfangswand 30 der Lagerhülse 29 in Eingriff gebracht werden kann. Dadurch wird die Handhabe 27 an der Lagerhülse 29 festlegbar, indem die Handhabe 27, wie in Fig. 2 gezeigt, auf die offene Stirnseite der Lagerhülse 29 aufgesetzt und die Rastkante 32 mit den Rastnasen 33 verrastet werden.

Zur Aufnahme der Zughülse 21 ist mittig in der verschlossenen Stirnfläche der Lagerhülse 29 mittig eine Öffnung vorgesehen, in der die Zughülse 21 angeordnet ist, wie in Fig. 2 erkennbar ist. Der Kragen 24 der Zughülse 21 liegt in der Fig. 2 gezeigten Stellung auf der der Handhabe zugewandten Oberfläche der Stirnwand der Lagerhülse 29 auf. In die Lageröffnung hinein erstrecken sich zwei diametral gegenüberliegende Vorsprünge 51 und 52, die in zwei Längsnuten 53 und 54 auf der Umfangsoberfläche der Zughülse 21 ragen. Die Längsnuten 53 und 54 verlaufen parallel zur Längsachse der Zughülse 21. Mit Hilfe der Führungsvorsprünge 51 und 52 und der Längsnuten 53 und 54 wird eine Verdrehsicherung der Zughülse 21 erreicht, so dass die Drehbewegung der Drehhülse 26 nicht zu einer Drehung sondern nur zu einer geradlinigen Verschiebung der Zughülse 21 führt. Wie sich aus Fig. 2 ergibt, ist damit die Zughülse 21 in dem Verbund aus der Handhabe 27 und Lagerhülse 29 axial verschiebbar aber verdrehsicher gehaltert. Wird nun die Handhabe 27 gegenüber der Lagerhülse 29 verdreht, dreht sich auch die Drehhülse 26 gegenüber der Zughülse 21, wodurch die Gleitvorsprünge 22 und 23 in den spiralförmigen Nuten 25 entlang laufen. Dadurch wird die Zughülse 21 in axialer Richtung in der Öffnung der Lagerhülse 29 verschoben.

Auf die Führungsvorsprünge 51 und 52 in der Lageröffnung und die Längsnuten 53 und 54 in der Zughülse 21 kann verzichtet werden, wenn durch die Gestaltung des Kegelstumpfs 19 und des Zylinderabschnitts 20 des Dichtungselements 16 sowie durch die großflächig Auflage der den Kegelstumpf aufnehmenden Stirnseite der Zughülse 21 auf dem ebenen Dichtungselementabschnitt 18 eine Verdrehsicherung der Zughülse 21 durch Reibung erreicht wird. Dazu muss aber das Dichtungselement 16 gegenüber der Lagerhülse 29 drehsicher festgelegt werden.

Auf der von der Handhabe abgewandten Oberfläche der verschlossenen Stirnseite der Lagerhülse 19 ist eine ringförmige erste Dichtlippe 34 konzentrisch zu der die Zughülse aufnehmenden Öffnung vorgesehen. Der Durchmesser der ersten Dichtlippe 34 entspricht dem Durchmesser der Umfangswand 14 des Flüssigkeitsbehälters 10. Wie in Fig. 2 erkennbar, wird dadurch erreicht, dass die erste Dichtlippe 34 das auf der Stirnseite der Umfangswand aufliegende Dichtungselement 16 derart an das Flüssigkeitsreservoir 10 anpresst, dass das Flüssigkeitsreservoir 10 abgedichtet wird. Daneben kann mit der ersten Dichtlippe 34 zusätzlich das Dichtungselement 16 drehsicher gegenüber dem Flüssigkeitsreservoir 10 und der Lagerhülse 29 festgelegt werden. Aufgrund der üblicherweise für das Dichtungselement einerseits und die übrigen Bauteile der erfindungsgemäßen Vorrichtung andererseits verwendeten Materialien ist keine übermäßig große Kraft aufzubringen, um die zuvor erwähnten Komponenten der erfindungsgemäßen Vorrichtung gegeneinander verdrehsicher festzulegen.

Bei dem hier beschriebenen vorteilhaften Ausführungsbeispiel erfolgt die Erzeugung der erforderlichen Kräfte zumindest teilweise durch eine Wechselwirkung der Handhabe 27 mit dem Gehäuse 35, in dem das Flüssigkeitsreservoir einstückig ausgebildet ist oder in das das Flüssigkeitsreservoir 10, wie in Fig. 2 gezeigt, eingesetzt ist. In diesem Fall liegt das in das Gehäuse eingesetzte Flüssigkeitsreservoir 10 mit dem umlaufenden Vorsprung 15 abschnittsweise auf einem Auflager 36 in dem Gehäuse 35 auf, das sich radial in das Innere des Gehäuses 35 erstreckt. Dadurch kann das Flüssigkeitsreservoir 10 aus dem Gehäuse 35 zu Reinigungszwecken einfach entnommen werden. Indem die Lagerung nur abschnittsweise erfolgt, entstehen Öffnungen für Umgebungsluft beim Einatmen der Patienten, was weiter unten noch genauer beschrieben wird.

In Fig. 2 nur teilweise erkennbar ist der Drehverschluss, der mit Hilfe der Handhabe 27 einerseits und dem Gehäuse 35 andererseits realisiert ist. Dargestellt sind lediglich Verschlussvorsprünge 62 und 63 am Gehäuse 35. Jedoch ergeben sich hinsichtlich des Ausgestaltung des Drehverschlusses keine besonderen Anforderungen im Hinblick auf die erfindungsgemäße Vorrichtung zur Erzeugung des Unterdrucks im Flüssigkeitsreservoir 10.

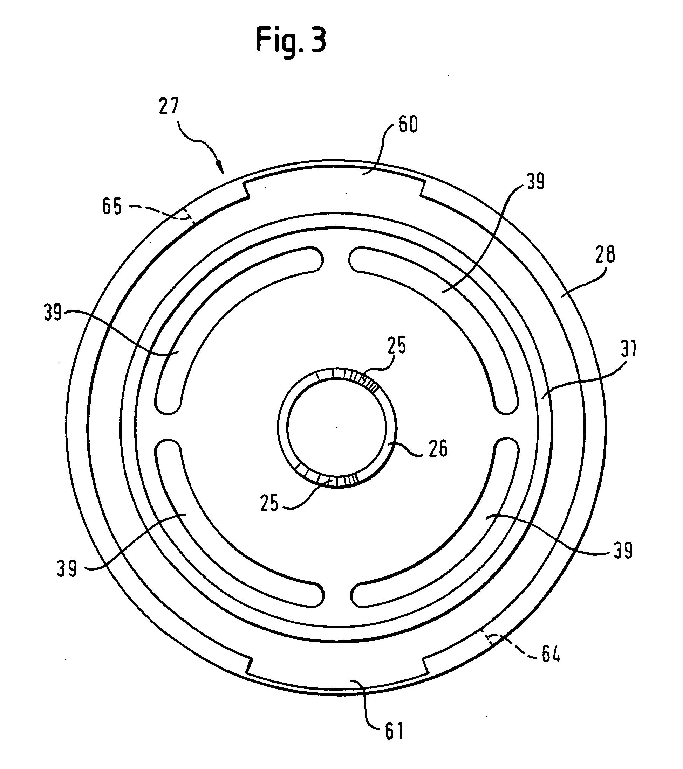

Unter Bezugnahme auf Fig. 3 wird eine besondere Ausgestaltung des Drehverschlusses beschrieben, bei der die Handhabe 27 und die Lagerhülse 29 aufeinander abgestimmt sind.

Fig. 3 zeigt eine Draufsicht auf die Handhabe 27 von der Seite, auf die die Lagerhülse 29 (in Fig. 3 nicht dargestellt) aufgesteckt wird. Wie in der Ansicht der Fig. 3 erkennbar, besitzt der zylindrische Teil 28 der Handhabe 27 Aussparungen 60 und 61 in die entsprechend gestaltete Verschlussvorsprünge 62 und 63 des Gehäuses (vgl. Fig. 2) einführbar sind. Wird die Handhabe 27 verdreht, gleiten die Vorsprünge 62 und 63 in nicht dargestellten Nuten des Bajonettverschlusses bis an die Anschläge 64 und 65; dies entspricht in Fig. 3 einer Drehung der dargestellten Handhabe 27 entgegen dem Uhrzeigersinn. In Fig. 3 ebenfalls erkennbar ist der zylindrische Rastring 31, dessen Rastkante 32 (vgl. Fig. 2) zur Verrastung der Handhabe 27 mit der Lagerhülse 29 dient. Mittig und konzentrisch zum Griffbereich 28 und Rastring 31 ist die Drehhülse 26 angeordnet, die einstückig mit der Handhabe 27 ausgebildet ist und bei der die spiralförmigen Nuten 25 erkennbar sind.

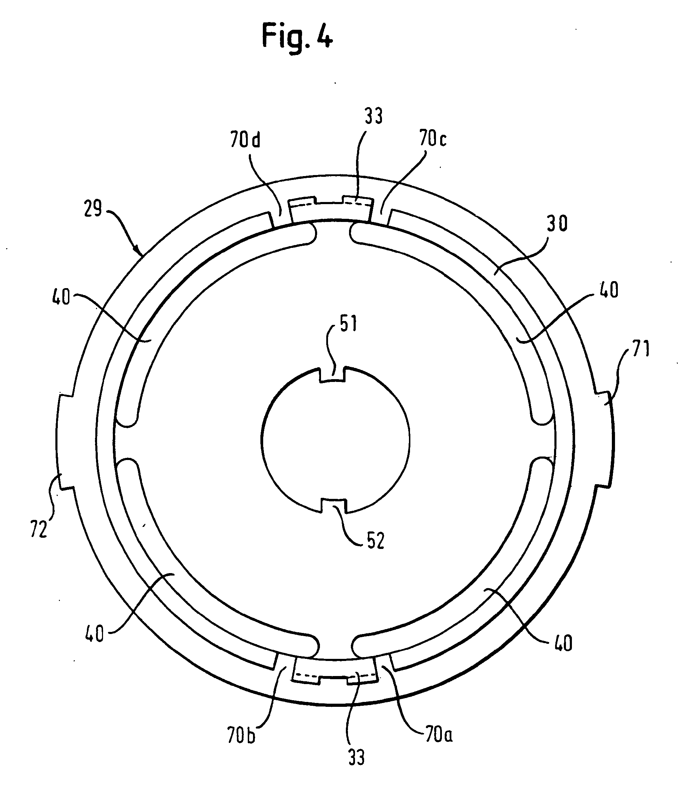

In Fig. 4 ist die Lagerhülse 29 gezeigt, und zwar von der Seite, die der Handhabe 27 im eingerasteten Zustand zugewandt ist. In die mittige Öffnung, in der die Zughülse 21 aufgenommen wird, ragen die beiden diametral angeordneten Führungsvorsprünge 51 und 52. Ferner ist die Umfangswand 30 erkennbar, die an vier Stellen 70a, 70b, 70c und 70d durchbrochen ist, so dass die elastischen Rastnasen 33 gebildet werden, die mit der Rastkante 32 am Rastring 31 der Handhabe 27 zusammenwirken. Durch die Aussparungen 70a - 70d sind die Rastnasen 33 federnd ausgebildet, so dass ein Verrasten durch Zurückweichen der Rastnasenelemente 33 erfolgen kann, wenn die Lagerhülse 29 in die Handhabe 27 eingesteckt wird. Senkrecht ausgerichtet zur Anordnung der Führungsvorsprünge 51 und 52 sind Arretiervorsprünge 71 und 72 diametral am Aussenumfang der Lagerhülse vorgesehen, die in entsprechende Ausnehmungen im Gehäuse 35 (nicht dargestellt) des Aerosoltherapiegeräts einführbar sind. Durch die Anordnung der Führungsvorsprünge 51 und 52, der Arretiervorsprünge 71 und 72 sowie der spiralförmigen Nuten 25 (vgl. Fig. 3) wird eine Grundstellung realisiert, die die Handhabe 27 gegenüber der Lagerhülse 29 einnimmt, wenn die beiden Elemente miteinander verrastet werden. Danach wird die Handhabe 27 gewissermaßen als Deckel auf das Gehäuse 35 aufgesteckt, so dass die Vorsprünge 62 und 63 des Gehäuses 35 in die Bajonettöffnungen 60 und 61 und gleichzeitig die Arretiervorsprünge 70 und 71 in die Aussparungen des Gehäuses 35 eingeführt werden. Die Aussparungen im Gehäuse 35 sowie die Arretiervorsprünge 71 und 72 der Lagerhülse 29 und die Vorsprünge 62 und 63 des Gehäuses 35 für die Bajonettöffnungen 60 und 61 sind rechtwinklig zueinander angeordnet. Dadurch wird erreicht, dass der Patient nach dem Aufsetzen der Handhabe auf das Gehäuse in einem Schritt eine Verriegelung des Bajonettverschlusses; eine den Unterdruck hervorrufende Bewegung der Dreh- und Zughülse und eine Abdichtung des Flüssigkeitsbehälters bewirkt.

Bei dem hier gezeigten Ausführungsbeispiel ist in vorteilhafter Weise das Dichtungselement 16 so ausgestaltet, dass neben den zuvor erwähnten Funktionen durch ein und dasselbe Dichtungselement ein Ausatemventil realisiert wird. Dazu ist das Dichtungselement 16 an dem auf der Stirnseite der Umfangswand 14 des Flüssigkeitsreservoirs 10 angeordneten Abschnitt mit einem Ventilabschnitt 37 ausgestattet, der das Dichtungselement 16 einstückig fortsetzt und der sich radial über die Umfangswand 14 des Flüssigkeitsreservoirs 10 hinaus erstreckt. Die Lagerhülse 29 umfasst eine zweite Dichtlippe 38, die konzentrisch zur ersten Dichtlippe 33 verläuft und deren Durchmesser größer ist als der der ersten Dichtlippe 34. Aufgrund der Festlegung des Dichtungselements 16 mit Hilfe der ersten Dichtlippe 34 und der Umfangswand 14 des Flüssigkeitsreservoirs 10 wird als zweiter Abschnitt der Ventilabschnitt 37 definiert, der in Zusammenwirken mit der zweiten Dichtlippe ein Rückschlagventil realisiert. Um eine Luftströmung zu ermöglichen, sind in der Handhabe 27 und in der Stirnwand der Lagerhülse 29 Öffnungen 39 bzw. 40 vorgesehen, durch die Umgebungsluft strömen kann (vgl. auch Fig. 3 und 4). Der verlängerte Abschnitt 37 des Dichtungselements 16 wird dann aus der Ruhelage, in der der verlängerte Abschnitt 37 an der zweiten Dichtlippe 38 anliegt, ausgelenkt.

Besonders vorteilhaft bei dieser Ausgestaltung ist der Umstand, dass das Dichtungselement 16 einstückig ausgebildet ist und sowohl die Funktion des Kolbens 16 und 18 für die Erzeugung des Unterdrucks im Flüssigkeitsreservoir 10 als auch die Funktion des flexiblen Ventilelements 37 für das Ausatemrückschlagventil übernimmt. Da das Dichtungselement 16 an der Zughülse 21 befestigt ist und letztere wiederum an der Lagerhülse 29, entsteht eine einheitlich handhabbare Komponentengruppe der erfindungsgemäßen Vorrichtung, die auch das Rückschlagventil umfasst.

Wie aus Fig. 3 und 4 in Verbindung mit Fig. 1 ersichtlich, ist ein Benutzer der erfindungsgemäßen Vorrichtung in der Lage, mit einem Griff den durch die Handhabe realisierten Deckel einer Aerosoltherapievorrichtung aufzusetzen und durch ein Verdrehen um die Mittellängsachse sowohl eine Verriegelung des Deckels als auch eine Verschiebung der Zughülse und damit die Erzeugung des Unterdrucks im Flüssigkeitsbehälter zu bewerkstelligen. Gleichzeitig befestigt der Benutzer das Ausatemrückschlagventil an der Aerosoltherapievorrichtung und dichtet den Flüssigkeitsbehälter ab.

## Patentansprüche

1. Aerosolgenerator, insbesondere für Aerosoltherapiegeräte mit
- einer Schwingmembran (5), der auf der einer Seite eine Flüssigkeit zuführbar ist und die in Schwingung versetzbar ist, um die Flüssigkeit durch Öffnungen in der Schwingmembran hindurch zu transportieren und um auf der anderen Seite der Schwingmembran die Flüssigkeit in Form eines Aerosol abzugeben,
- einem Flüssigkeitsreservoir (10) für die Aufnahme der Flüssigkeit, das zur Zuführung der Flüssigkeit mit der Schwingmembran (5) in Verbindung steht,
- einem Dichtungselement (16), das an einer Öffnung des Flüssigkeitsreservoirs (16) angeordnet ist, um die Öffnung gasdicht zu verschließen, und
- einem Zugelement (21), das mit dem Dichtungselement (16) derart verbunden ist, dass eine Bewegung des Zugelements (21) eine Bewegung zumindest eines Abschnitts (18) des Dichtungselements (16) bewirkt, wodurch ein Unterdruck in dem Flüssigkeitsreservoir (10) erzeugt wird.

2. Aerosolgenerator nach Anspruch 1 oder 2, **dadurch gekennzeichnet, dass** ein Drehelement (26) vorgesehen ist, das mit dem Zugelement (21) derart verbunden ist, dass eine Drehung des Drehelements (26) um die Längsachse eine im wesentlichen geradlinige Bewegung des Zugelements (21) bewirkt.

3. Aerosolgenerator nach einem der Ansprüche 1 oder 2, **dadurch gekennzeichnet, dass** das Zugelement eine Zughülse (21) und das Drehelement eine Drehhülse (26) ist.

4. Aerosolgenerator nach einem der Ansprüche 1 bis 3, **dadurch gekennzeichnet, dass** das Dichtelement (16) aus einem elastischen Material besteht, so dass das Dichtelement zumindest bereichsweise verformbar ist.

5. Aerosolgenerator nach einem der Ansprüche 1 bis 4, **dadurch gekennzeichnet, dass** das Zugelement (10) und das Dichtungselement (16) einstückig aber aus unterschiedlichen Materialien gefertigt sind.

6. Aerosolgenerator nach einem der Ansprüche 1 bis 5, **dadurch gekennzeichnet, dass** das Dichtungselement (16) einen ebenen Abschnitt (18) aufweist, an dem ein Vorsprung (19, 20) vorgesehen ist, und dass das Zugelement (21) eine Öffnung aufweist, in der der Vorsprung (19, 20) zur Verbindung des Zugelements (21) mit dem Dichtungselement (16) angeordnet ist.

7. Aerosolgenerator nach Anspruch 6, **dadurch gekennzeichnet, dass** der Vorsprung einen kegelförmigen Abschnitt (19) und einen zylindrischen Abschnitt (20) umfasst und dass die Öffnung in der Zughülse (21) an den zylindrischen Abschnitt angepasst ist.

8. Aerosolgenerator nach einem der Ansprüche 1 bis 7, **dadurch gekennzeichnet, dass** das Dichtungslement (16) einen konischen Wandabschnitt (17) aufweist, der von dem ebenen Wandabschnitt (18) abgeschlossen wird.

9. Aerosolgenerator nach Anspruch 8, **dadurch gekennzeichnet, dass** zwischen dem ebenen Wandabschnitt (18) und dem konischen Wandabschnitt (17) ein den ebenen Wandabschnitt umlaufender Bereich derart ausgestaltet ist, dass der ebene Wandabschnitt (18) von dem konischen Wandabschnitt (16) beabstandet ist.

10. Aerosolgenerator nach einem der Ansprüche 3 bis 9, **dadurch gekennzeichnet, dass** die Zughülse (21) ein erster einseitig offener Zylinder ist, der an der offenen Stirnseite zumindest zwei Gleitvorsprünge (22, 23) aufweist, die sich radial in das Innere des Zylinders erstrecken, und dass die Drehhülse (26) ein zweiter einseitig offener Zylinder ist, der auf der äußeren Umfangsoberfläche zumindest zwei spiralförmig verlaufende Nuten (25) aufweist und der in der Zughülse (21) derart angeordnet ist, dass die Gleitvorsprünge (22, 23) jeweils in einer spiralförmig verlaufenden Nut (25) angeordnet sind.

11. Aerosolgenerator nach Anspruch 10, **dadurch gekennzeichnet, dass** die Drehhülse (26) einstückig mit einer Handhabe (27) ausgebildet ist, die ein Verdrehen der Drehhülse (26) von Hand unterstützt.

12. Aerosolgenerator nach Anspruch 11, **dadurch gekennzeichnet, dass** die Handhabe (27) im wesentlicher ein flacher Zylinder oder Kegelstumpf mit einem Griffbereich (28) ist.

13. Aerosolgenerator nach Anspruch 11 oder 12, **dadurch gekennzeichnet, dass** die Handhabe (27) einen Rastring (31) mit einer Rastkante (32) aufweist und dass eine Lagerhülse (29) vorgesehen ist, die Rastnasen (33) zum Verrasten der Lagerhülse (29) an der Rastkante (32) der Handhabe (27) aufweist und in der eine Öffnung ausgebildet ist, in der die Zughülse (21) angeordnet ist.

14. Aerosolgenerator nach Anspruch 13, **dadurch gekennzeichnet, dass** die Zughülse (21) eine umlaufende Kante (24) aufweist, die in zumindest einer Stellung auf der Lagerhülse (24) auflegt.

15. Aerosolgenerator nach Anspruch 13 oder 14, **dadurch gekennzeichnet, dass** die Lagerhülse (29) eine erste Dichtlippe (34) zum Anpressen des Dichtungselements (26) an eine Oberfläche des Flüssigkeitsreservoirs (10) aufweist, die die von dem Dichtungselement verschlossene Öffnung des Flüssigkeitsreservoirs (10) umgibt.

16. Aerosolgenerator nach Anspruch 15, **dadurch gekennzeichnet, dass** die Lagerhülse (19) eine zweite Dichtlippe (28) aufweist, die konzentrisch zur ersten Dichtlippe (24) angeordnet ist und dass das Dichtungselement (6) zur Realisierung eines Einatemventils einen Ventilabschnitt (27) aufweist, der sich zwischen der ersten und der zweiten Dichtlippe (24, 28) erstreckt.

17. Aerosolgenerator nach einem der vorangegangenen Ansprüche 3 bis 16, **dadurch gekennzeichnet, dass** die Zughülse (21) in der Umfangsoberfläche zumindest eine Längsnut (53, 54) aufweist, die mit zumindest einem Führungsvorsprung (51, 52) zur Verdrehsicherung der Zughülse (21) zusammenwirkt.

18. Aerosolgenerator nach Anspruch 17, **dadurch gekennzeichnet, dass** der Führungsvorsprung (53, 54) in der Öffnung der Lagerhülse (29) angeordnet ist.

19. Aerosolgenerator nach einem der vorangegangenen Ansprüche, **dadurch gekennzeichnet, dass** die Schwingmembran (5) in einer Stirnfläche des Flüssigkeitsreservoirs (10) angeordnet ist.

20. Aerosoltherapiegerät mit einem Aerosolgenerator nach einem der vorangegangenen Ansprüche.

21. Aerosoltherapiegerät nach Anspruch 20, **dadurch gekennzeichnet, dass** die Handhabe (27) als Deckel des Aerosoltherapiegeräts ausgestaltet ist und mit dem Gehäuse (35) des Aerosoltherapiegeräts über einen Drehverschluss (28, 60, 61, 62, 63) verbindbar ist.

22. Aerosoltherapiegerät nach Anspruch 20 oder 21, **dadurch gekennzeichnet, dass** die Lagerhülse (29) zumindest einen Vorsprung (71, 72) aufweist, der mit einer Rastnut im Gehäuse des Aerosoltherapiegeräts zur Verdrehsicherung der Lagerhülse (29) zusammenwirkt.

## Claims

1. Aerosol generator, in particular for aerosol treatment devices having
- a vibrating membrane (5), to which a liquid can be supplied on the one side and which can be vibrated in order to transport the liquid through openings in the vibrating membrane and in order to release the liquid in the form of an aerosol on the other side of the vibrating membrane,
- a liquid reservoir (10) for receiving the liquid, which is connected to the vibrating membrane (5) for supplying the liquid,
- a sealing element (16), which is arranged on an opening of the liquid reservoir (16) in order to close the opening in gastight manner, and
- a tension element (21), which is connected to the sealing element (16) such that a movement of the tension element (21) effects a movement of at least one section (18) of the sealing element (16), as a result of which negative pressure is produced in the liquid reservoir (10).

2. Aerosol generator according to claim 1 or 2, **characterised in that** a rotary element (26) is provided which is connected to the tension element (21) such that a rotation of the rotary element (26) about the longitudinal axis effects an essentially straight-line movement of the tension element (21).

3. Aerosol generator according to one of claims 1 or 2, **characterised in that** the tension element is a tension sleeve (21) and the rotary element is a rotary sleeve (26).

4. Aerosol generator according to one of claims 1 to 3, **characterised in that** the sealing element (16) consists of a resilient material, so that the sealing element can be deformed at least in some regions.

5. Aerosol generator according to one of claims 1 to 4, **characterised in that** the tension element (10) and the sealing element (16) are made to be integral but from different materials.

6. Aerosol generator according to one of claims 1 to 5, **characterised in that** the sealing element (16) has a flat section (18), on which a projection (19, 20) is provided, and **in that** the tension element (21) has an opening, in which the projection (19, 20) is arranged for connection of the tension element (21) to the sealing element (16).

7. Aerosol generator according to claim 6, **characterised in that** the projection comprises a conical section (19) and a cylindrical section (20) and **in that** the opening in the tension sleeve (21) is adapted to the cylindrical section.

8. Aerosol generator according to one of claims 1 to 7, **characterised in that** the sealing element (16) has a conical wall section (17), which is sealed off by the flat wall section (18).

9. Aerosol generator according to claim 8, **characterised in that** a region surrounding the flat wall section is designed between the flat wall section (18) and the conical wall section (17), such that the flat wall section (18) is spaced from the conical wall section (16).

10. Aerosol generator according to one of claims 3 to 9, **characterised in that** the tension sleeve (21) is a first cylinder which is open on one side, which has at least two sliding projections (22, 23) on the open end-face side which extend radially into the interior of the cylinder, and **in that** the rotary sleeve (26) is a second cylinder which is open on one side, which has at least two helically running grooves (25) on the outer peripheral surface and which is arranged in the tension sleeve (21) such that the sliding projections (22, 23) are arranged in each case in a helically running groove (25).

11. Aerosol generator according to claim 10, **characterised in that** the rotary sleeve (26) is designed to be integral with a handle (27), which assists rotation of the rotary sleeve (26) by hand.

12. Aerosol generator according to claim 11, **characterised in that** the handle (27) is essentially a flat cylinder or frustum with a gripping region (28).

13. Aerosol generator according to claim 11 or 12, **characterised in that** the handle (27) has a locking ring (31) with a locking edge (32) and **in that** a bearing sleeve (29) is provided which has locking noses (33) for locking the bearing sleeve (29) to the locking edge (32) of the handle (27) and in which an opening is formed, in which the tension sleeve (21) is arranged.

14. Aerosol generator according to claim 13, **characterised in that** the tension sleeve (21) has an annular edge (24), which rests on the bearing sleeve (24) in at least one position.

15. Aerosol generator according to claim 13 or 14, **characterised in that** the bearing sleeve (29) has a first sealing lip (34) for pressing the sealing element (26) on a surface of the liquid reservoir (10), which surrounds the opening of the liquid reservoir (10) closed by the sealing element.

16. Aerosol generator according to claim 15, **characterised in that** the bearing sleeve (19) has a second sealing lip (28), which is arranged concentrically to the first sealing lip (24) and **in that** the sealing element (6) has a valve section (27) to realise an inhaling valve, which valve section (27) extends between the first and the second sealing lip (24, 28).

17. Aerosol generator according to one of the preceding claims 3 to 16, **characterised in that** the tension sleeve (21) has in the peripheral surface at least one longitudinal groove (53, 54) which cooperates with at least one guide projection (51, 52) for protection against torsion of the tension sleeve (21).

18. Aerosol generator according to claim 17, **characterised in that** the guide projection (53, 54) is arranged in the opening of the bearing sleeve (29).

19. Aerosol generator according to one of the preceding claims, **characterised in that** the vibrating membrane (5) is arranged in one end-face surface of the liquid reservoir (10).

20. Aerosol treatment device having an aerosol generator according to one of the preceding claims.

21. Aerosol treatment device according to claim 20, **characterised in that** the handle (27) is designed as a cover of the aerosol treatment device and can be connected to the housing (35) of the aerosol treatment device via a rotary closure (28, 60, 61, 62, 63).

22. Aerosol treatment device according to claim 20 or 21, **characterised in that** the bearing sleeve (29) has at least one projection (71, 72), which cooperates with a locking groove in the housing of the aerosol treatment device for protection against torsion of the bearing sleeve (29).

## Revendications

1. Dispositif de production d'aérosol, en particulier pour :
des appareils de thérapie par aérosol, comprenant :
- une membrane vibrante (5), à laquelle, sur une face, peut être amenée un liquide et qui est susceptible d'être mise en vibration, pour transporter le liquide à travers des ouvertures ménagées dans la membrane oscillante et pour délivrer le liquide sur l'autre face de la membrane oscillante, sous la forme d'un aérosol,
- un réservoir à liquide (10) pour recevoir le liquide, le réservoir étant relié à la membrane oscillante (5) pour l'amenée du liquide,
- un élément d'étanchéité (16) disposé sur une ouverture du réservoir à liquide (10), pour fermer l'ouverture de façon étanche au gaz, et
- un élément de traction (21), relié à l'élément d'étanchéité (16) de manière qu'un déplacement de l'élément de traction (21) provoque un déplacement au moins d'un tronçon (18) de l'élément d'étanchéité (16), faisant qu'une pression négative est produite dans le réservoir à liquide (10).

2. Dispositif de production d'aérosol selon la revendication 1 ou 2, **caractérisé en ce qu'**un élément rotatif (26) est prévu, relié à l'élément de traction (21) de manière qu'une rotation de l'élément rotatif (26) autour de l'axe longitudinal provoque un déplacement sensiblement rectiligne de la part de l'élément de traction (21).

3. Dispositif de production d'aérosol selon l'une des revendications 1 ou 2, **caractérisé en ce que** l'élément de traction est une douille de traction (21) et l'élément rotatif est une douille rotative (26).

4. Dispositif de production d'aérosol selon l'une des revendications 1 à 3, **caractérisé en ce que** l'élément d'étanchéité (16) est composé d'un matériau élastique, de manière que l'élément d'étanchéité soit déformable au moins par zones.

5. Dispositif de production d'aérosol selon l'une des revendications 1 à 4, **caractérisé en ce que** l'élément de traction (10) et l'élément d'étanchéité (16) sont fabriqués d'une seule pièce, mais en des matériaux différents.

6. Dispositif de production d'aérosol selon l'une des revendications 1 à 5, **caractérisé en ce que** l'élément d'étanchéité (16) présente un tronçon (18) plan, sur lequel est prévue une saillie (19, 20), et que l'élément de traction (21) présente une ouverture, dans laquelle la saillie (19, 20) est disposée pour assurer la liaison de l'élément de traction (21) à l'élément d'étanchéité (16).

7. Dispositif de production d'aérosol selon la revendication 6, **caractérisé en ce que** la saillie comprend un tronçon (19) à forme conique et un tronçon (20) à forme cylindrique, et **en ce que** l'ouverture ménagée dans la douille de traction (21) est adaptée au tronçon à forme cylindrique.

8. Dispositif de production d'aérosol selon l'une des revendications 1 à 7, **caractérisé en ce que** l'élément d'étanchéité (16) présente un tronçon de paroi (17) à forme conique, fermé par le tronçon de paroi (18) plan.

9. Dispositif de production d'aérosol selon la revendication 8, **caractérisé en ce que**, entre le tronçon de paroi (18) plan et le tronçon de paroi (17) conique est réalisée une zone faisant le pourtour du tronçon de paroi plan, de manière que le tronçon de paroi (18) plan soit maintenu à distance du tronçon de paroi (16) conique.

10. Dispositif de production d'aérosol selon l'une des revendications 3 à 9, **caractérisé en ce que** la douille de traction (21) est un premier cylindre ouvert sur un côté, présentant sur la face frontale ouverte au moins deux saillies de glissement (22, 23) qui s'étendent radialement à l'intérieur du cylindre, et **en ce que** la douille de rotation (26) est un deuxième cylindre ouvert sur un côté, présentant sur la surface périphérique extérieure au moins deux rainures (26) s'étendant en forme de spirale et qui est disposée dans la douille de traction (21), de manière que les saillies de glissement (22, 23) soient chacune disposées dans une rainure (25) s'étendant en forme de spirale.

11. Dispositif de production d'aérosol selon la revendication 10, **caractérisé en ce que** la douille de rotation (26) est réalisée d'une seule pièce avec une manette (27), favorisant manuellement une rotation de la douille de rotation (26).

12. Dispositif de production d'aérosol selon la revendication 11, **caractérisé en ce que** la manette (27) est essentiellement un cylindre ou un tronc de cône plat muni d'une zone de saisi (28).

13. Dispositif de production d'aérosol selon la revendication 11 ou 12, **caractérisé en ce que** la manette (27) présente une bague d'encliquetage (31) avec une arête d'encliquetage (32), et **en ce qu'**une douille de palier (29) est prévue, présentant des ergots d'encliquetage (33) pour l'encliquetage de la douille de palier (29) sur l'arête d'encliquetage (32) de la manette (27) et douille dans laquelle est formée une ouverture, dans laquelle la douille de traction (21) est disposée.

14. Dispositif de production d'aérosol selon la revendication 13, **caractérisé en ce que** la douille de traction (21) présente une arête (24) de pourtour, reposant en au moins une position sur la douille de palier (24).

15. Dispositif de production d'aérosol selon la revendication 13 ou 14, **caractérisé en ce que** la douille de palier (29) présente une première lèvre d'étanchéité (34) pour pressage de l'élément d'étanchéité (26) sur une surface du réservoir à liquide (10) qui entoure l'ouverture, fermée par l'élément d'étanchéité, du réservoir à liquide (10).

16. Dispositif de production d'aérosol selon la revendication 15, **caractérisé en ce que** la douille de palier (29) présente une deuxième lèvre d'étanchéité (28) disposée concentriquement par rapport à la première lèvre d'étanchéité (24), et **en ce que** l'élément d'étanchéité (6) présente, pour réaliser une soupape d'inhalation, un tronçon de soupape (27) s'étendant entre la première et la deuxième lèvres d'étanchéité (24, 28).

17. Dispositif de production d'aérosol selon l'une des revendications 3 à 16 précédentes, **caractérisé en ce que** la douille de traction (21) présente, dans la surface périphérique, au moins une rainure longitudinale (53, 54), coopérant avec au moins une saillie de guidage (51, 52) pour assurer la sécurité en rotation de la douille de traction (21).

18. Dispositif de production d'aérosol selon la revendication 17, **caractérisé en ce que** la saillie de guidage (53, 54) est disposée dans l'ouverture de la douille de palier (29).

19. Dispositif de production d'aérosol selon l'une des revendications précédentes, **caractérisé en ce que** la membrane vibrante (5) est disposée dans une face frontale du réservoir à liquide (10).

20. Appareil de thérapie par aérosol, avec un dispositif de production d'aérosol selon l'une des revendications précédentes.

21. Appareil de thérapie par aérosol selon la revendication 20, **caractérisé en ce que** la manette (27) est réalisée en tant que couvercle de l'appareil de thérapie par aérosol et est susceptible d'être reliée au boîtier (35) de l'appareil de thérapie par aérosol, par l'intermédiaire d'une fermeture rotative (28, 60, 61, 62, 63).

22. Appareil de thérapie par aérosol selon la revendication 20 ou 21, **caractérisé en ce que** la douille de palier (29) présente au moins une saillie (71, 72), coopérant avec une rainure d'encliquetage ménagée dans le boîtier de l'appareil de thérapie par aérosol, afin d'assurer la sécurité en rotation de la douille de palier (29).
